(19) Europäisches Patentamt
European Patent Office
Office européen des brevets

(11) **EP 1 571 226 B1**

(12) **EUROPEAN PATENT SPECIFICATION**

(45) Date of publication and mention
of the grant of the patent:
**27.04.2011 Bulletin 2011/17**

(51) Int Cl.:
*C12Q 1/68* (2006.01)  *A01K 1/03* (2006.01)
*G06F 19/00* (2011.01)

(21) Application number: **05005342.0**

(22) Date of filing: **10.03.2000**

(54) **Gene chip technology for determining memory genes**

Genchiptechnologie zur Bestimmung von Gedächtnisgenen

Technologie des puces à adn pour déterminer des gènes de mémoire

(84) Designated Contracting States:
**AT BE CH CY DE DK ES FI FR GB GR IE IT LI LU MC NL PT SE**

(30) Priority: **10.03.1999 US 124085 P**

(43) Date of publication of application:
**07.09.2005 Bulletin 2005/36**

(62) Document number(s) of the earlier application(s) in accordance with Art. 76 EPC:
**00914897.4 / 1 196 627**

(73) Proprietors:
• **COLD SPRING HARBOR LABORATORY**
Cold Spring Harbor, NY 11724 (US)
• **Emory University**
Atlanta, GA 30322 (US)
• **F. Hoffmann-La Roche AG**
4070 Basel (CH)

(72) Inventors:
• **Tully, Timothy P.**
Cold Spring Harbor, NY 11724 (US)
• **Dubnau, Joshua I.**
Huntington Station, NY 11746 (US)
• **Davis, Michael**
Stone Mountain, GA 30087 (US)
• **Mous, Jan**
4304 Giebenach (CH)
• **Certa, Ulrich**
4123 Allschwil (CH)

(74) Representative: **Kirkham, Nicholas Andrew et al**
**Graham Watt & Co LLP**
**St Botolph's House**
**7-9 St Botolph's Road**
**Sevenoaks**
**Kent TN13 3AJ (GB)**

(56) References cited:
**WO-A-96/11270      WO-A-97/12976
WO-A-98/49342**

• **SILVA A J ET AL: "CREB AND MEMORY"** ANNUAL REVIEW OF NEUROSCIENCE, ANNUAL REVIEWS INC., PALO ALTO, CA, US, vol. 21, 1998, pages 127-148, XP000900517 ISSN: 0147-006X
• **GUZOWSKI J F ET AL: "ANTISENSE OLIGODEOXYNUCLEOTIDE-MEDIATED DISRUPTION OF HIPPOCAMPAL CAMP RESPONSE ELEMENT BINDING PROTEIN LEVELS IMPAIRS CONSOLIDATION OF MEMORY FOR WATER MAZE TRAINING"** PROCEEDINGS OF THE NATIONAL ACADEMY OF SCIENCES OF USA, NATIONAL ACADEMY OF SCIENCE. WASHINGTON, US, vol. 94, March 1997 (1997-03), pages 2693-2698, XP000934313 ISSN: 0027-8424
• **TULLY T ET AL: "INDEPENDENT MEMORIES IN DROSOPHILA AFTER PAVLOVIAN CONDITIONING"** SOCIETY FOR NEUROSCIENCE ABSTRACTS, SOCIETY FOR NEUROSCIENCE, US, vol. 19, no. 1/3, 1993, page 1066, XP000566270 ISSN: 0190-5295
• **CAVALLARO S ET AL: "Late memory-related genes in the hippocampus revealed by RNA fingerprinting"** PROC. NATL. ACAD SCI USA, vol. 94, September 1977 (1977-09), pages 9669-9673, XP002178412

- BOURTCHOULADZE R ET AL: "Different training procedures recruit either one or two critical periods for contectual memory consolidation, each of which requires protein synthesis and PKA" LEARNING AND MEMORY, vol. 5, no. 4-5, 1998, pages 365-74, XP001024521
- KOGAN J ET AL: "Spaced training induces normal long-term memory in CREB mutant mice" CURRENT BIOLOGY, vol. 7, no. 1, 1997, pages 1-11, XP001024551
- PEDREIRA M ET AL: "Massed and spaced training build up different components of long-term habituation in the crab Chasmagnathus" ANIMAL LEARNING AND BEHAVIOUR, vol. 26, no. 1, 1998, pages 34-45, XP001024597

**Description**

BACKGROUND OF THE INVENTION

An attribute that many organisms, including humans, possess is memory of past events. This attribute has been studied for many decades with much information now available that explains many of its ramifications. For example, two basic types of memory have been identified: transcription-independent memory, which includes short term memory, and transcription-dependent memory, which includes long term memory.

An heretofore relatively unknown aspect of memory is the identity of genes that contribute to its manifestation. The identity of the genes that contribute to memory formation is just beginning to be explored. Identification of genes associated with memory formation would provide (a) a genetic epidemiology of cognitive dysfunction, (b) diagnostic tools for individuals carrying different allelic forms of these genes (associative with different performance levels for particular forms of cognition) and (c) new targets for drug discovery ultimately to ameliorate various forms of cognitive dysfunction (and particular drugs could be matched to particular forms of cognitive dysfunction by the diagnostic tests). Thus, it would be useful to have techniques available that would identify the genes that are associated with memory formation.

Cavallaro S et al., PNAS, 94:9669-9673 (1997) discloses the use of RNA fingerprinting to identify memory related genes. Tully et al., Society for Neuroscience abstracts, vol. 19, no. 1/3:1066 (1993) discloses that independent memories are induced in Drosophila after pavlovian conditioning using massed and spaced training protocols. WO9849342 discloses the use of microarrays for preparing a standard diagnostic gene transcript pattern.

SUMMARY OF THE INVENTION

The present invention is related to Applicants' discovery that the differential effects on memory formation produced by certain experimental protocols can be used to identify genes involved in transcription-dependent memory formation, particularly long term memory formation. The significant difference between any two experimental protocols is in the induction of transcription-dependent memory. The significant difference between any particular two experimental protocols to be compared is the induction of transcription-dependent memory in the experimental group and the absence of transcription-dependent memory in the control group.

Transcription-independent memory includes various "memory phases", such as short-term memory, intermediate-(or middle-) term memory and (in flies) anesthesia-resistant memory. In common to these forms is that pharmacological inhibitors of RNA transcription do not disrupt these memories. Transcription-dependent memory usually is referred to as long-term memory and inhibitors of RNA synthesis block its appearance.

As a result, Applicants' invention relates to methods of identifying a gene or genes involved in transcription-dependent memory (particularly long term memory) comprising (a) training non-human animals (particularly non-human mammals, other vertebrates and invertebrates) as set forth in claim 1.

The experimental conditions of step (a) and step (i) constitute an (experimental) treatment pair. The significant difference between the experimental conditions of step (a) and step (i) is in the induction of transcription-dependent memory.

As used herein, a control animal is an animal that is of the same species as, and otherwise comparable to (e.g., similar age, sex), the animal that is trained under conditions sufficient to induce transcription-dependent memory formation in that animal.

In a particular embodiment, RNA is extracted from the amygdala of trained or control animals. In another embodiment, RNA is extracted from the hippocampus of trained or control animals. In still another embodiment, the signal from hybridized probes is amplified, prior to detection. In another embodiment, a statistical comparison is made (performed, conducted) between the signal detected in step (e) and the signal detected in a control that is obtained by training control animals under conditions sufficient to induce transcription-independent memory but not transcription-dependent memory.

Transcription-dependent memory can be induced using specific experimental conditions. In one embodiment, transcription-dependent memory is induced in a non-human animal using a spaced training protocol for the fear-potentiated startle response. Transcription-dependent memory can be induced in a non-human animal using a shuttle-box avoidance protocol. In another embodiment, transcription-dependent memory is induced in a non-human animal using a contextual fear conditioning protocol.

The invention also relates to a method of identifying a gene or genes involved in transcription-dependent memory in Drosophila.

As used herein, a control Drosophila is a Drosophila that is of the same species as, and otherwise comparable to, the Drosophila that is trained under conditions sufficient to induce transcription-dependent memory in that Drosophila.

In one embodiment of the method of identifying a gene or genes involved in transcription-dependent memory in Drosophila, the DNA probes are labeled with a fluorescent marker and the signal is detected using a fluorescence assay. In a particular embodiment, the signal from hybridized probes is amplified prior to detection. In the invention, a statistical comparison is performed between the signal detected in step (e) and the signal detected in a control that is obtained by training control Drosophila under conditions sufficient to induce transcription-independent memory but not transcription-dependent memory.

Transcription-dependent memory can be induced in Drosophila using a spaced training protocol (e.g., spaced training of olfactory Pavlovian conditioning). Transcription-independent memory can be induced in Drosophila using a massed training protocol (massed training of olfactory Pavlovian conditioning).

A statistically significant difference in transcript level for a specific gene between animals trained under conditions sufficient to induce transcription-dependent memory and control animals trained under appropriate conditions that are not sufficient to induce transcription-dependent memory identifies that gene as a candidate memory gene (CMG). A statistically significant difference in transcript level between spaced- and massed-trained groups for a specific gene identifies that gene as a candidate memory gene.

A statistically significant difference in transcript level for a specific gene between animals trained under conditions sufficient to induce transcription-dependent memory and naïve (untrained) control animals identifies that gene as a candidate plasticity gene (CPG). In a particular embodiment, a statistically significant difference in transcript level between spaced-trained and untrained groups for a specific gene identifies that gene as a candidate plasticity gene.

BRIEF DESCRIPTION OF THE DRAWINGS

Figure 1 is a schematic diagram of a training protocol described in Tully et al., Cell, 79:35-47 (1994)), which produces Pavlovian olfactory learning in flies.

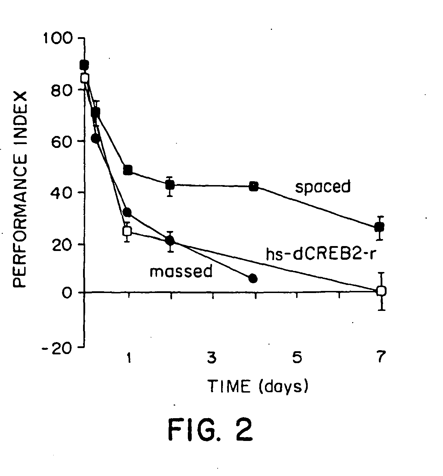

Figure 2 is a graphic representation of results showing memory retention after spaced or massed training in normal (wild type) flies or spaced training in transgenic hs-CREB2-r flies after induced expression of CREB repressor (see Yin et al., Cell, 79:49-58 (1994)). Learning and early memory (cycloheximide insensitive) are normal in transgenic flies. The additional (protein synthesis-dependent LTM) memory normally produced by spaced training is blocked in transgenic flies. This comparison reveals that the only difference between spaced and massed training is the appearance of a transcription-dependent memory after the former.

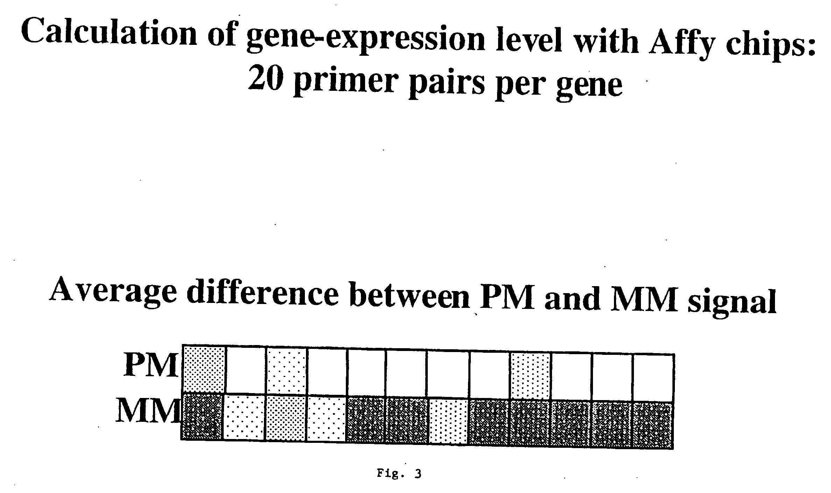

Figure 3 is a schematic diagram showing the average difference between signal detected for a specific DNA oligonucleotide probe perfectly matched (PM) (complementary) to a specific section of a specific gene and signal detected for that probe mismatched (MM) to that section of the gene as a result of the introduction of a nucleotide sequence error (mutation) in that section of the gene. The average difference between PM and MM pairs, and usually for 20 pairs per gene, is determined by Affymetrix design software analysis (Affymetrix, Inc., Santa Clara, CA). The squares represent microsequences on a microarray chip.

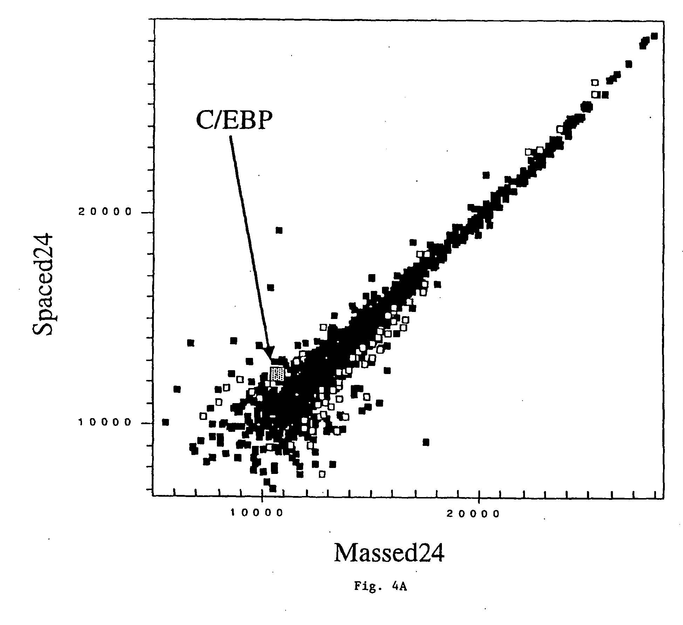

Figure 4A is a scatterplot representation (spaced versus massed) of the mean signal (mean transformed normalized difference) from an N = 10 chips, each hybridized with DNA probes made from RNA extracted from the heads of normal Drosophila exposed 24 hours earlier to either spaced or massed training. Each square represents a specific Drosophila gene, 1542 of which are contained on each chip. The candidate memory genes are identified by the lighter shaded squares. The location of the C/EBP gene in the plot is indicated in the figure.

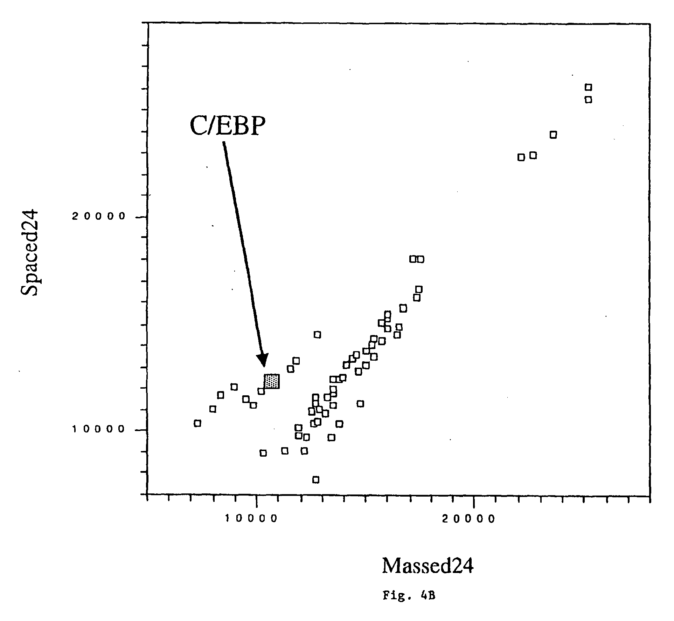

Figure 4B is a scatterplot representation (spaced verus massed) showing the statistically significant values from Figure 4A. Each square represents a specific Drosophila gene, 1542 of which are contained on each chip. The location of the C/EBP gene in the plot is indicated in the figure.

Figure 5 is a bar graph showing the results from a quantitative polymerase chain reaction (QPCR) experiment. The results confirm the differential effect of spaced versus massed training on the C/EBP gene.

Figure 6 is a bar graph showing the effect of an intertrial interval (ITI) between fear conditioning training trials in rats on subsequent long-term memory. The results define massed- and spaced-trained protocols and show that memory of fear-potentiated startle is better after spaced-training than after massed-training.

Figure 7 is a bar graph showing the effect on long term memory of overexpression of CREB activator in the amygdala of rats. The results show that overexpression of CREB activator in the amygdala enhances (increases) memory of fear-potentiated startle in rats after massed training.

Figure 8 depict molecular maps of the *adf1* genomic region.

DETAILED DESCRIPTION OF THE INVENTION

To produce a specific "long-term memory," an animal is subjected to a specific training protocol under controlled, experimental conditions. In Pavlovian conditioning procedures, for instance, two specific stimuli are presented in temporal contiguity to produce "associative learning and memory." One of the two stimuli is designated a "conditioned stimulus" (CS) and the other is designated an "unconditioned stimulus" (US). The US usually is a natural reinforcer that elicits a "unconditioned response" (UR) before training in a "reflexive" manner. With CS-US pairing, a "conditioned response" (CR) begins to appear in response to the CS before (or in the absence of) presentation of the US. After a CR to a specific CS-US pairing is "learned", memory formation thereafter begins.

Memory formation of this specific, experimental experience can exist in two general forms: a transcription-independent form and a transcription-dependent form. The former includes various "memory phases," such as short-term memory, intermediate-(or middle-) term memory and (in flies) anesthesia-resistant memory. In common to these forms is that pharmacological inhibitors of RNA transcription do not disrupt these memories. The latter form usually is referred to as long-term memory and inhibitors of RNA synthesis block its appearance.

In animal models, various experimental treatments, such as gene mutation, pharmacological blockade, anatomical lesion or specific training protocols, can affect one or more of these types of memories. In particular, some experimental treatments yield normal amounts of transcription-independent memory but do not yield transcription-dependent memory. Such observations constitute the basis of informative DNA chip comparisons. In general, a comparison is made between two experimental protocols; one (experimental group) that is sufficient to induce both transcription-independent and transcription-dependent memories and one that yields only transcription-independent memory (control group). Any detectable differences in transcript levels between these two protocols then can be attributed specifically to a transcription-dependent memory of the experimentally induced learning. These transcripts are referred to herein as "Candidate Memory Genes" (CMGs).

Although experimental conditions are controlled to induce a specific type of learning, other experimentally uncontrolled forms of learning also may take place. Thus, although a control group may not yield transcription-dependent memory of the specific experimental task, it nevertheless may yield a transcription-dependent memory of an uncontrolled learning experience. One type of such experience is the potential "nonassociative" forms of learning that occur in response to only the CS or US (alone), or in response to CS-US presentations that are not paired temporally (which is the key requirement for "associative learning"). Hence, transcription-dependent "nonspecific" memories may exist in control groups, as defined above. This observation gives rise to a broader class of transcripts involved with "nonspecific" learning, which we refer to as Candidate Plasticity Genes (CPGs). DNA chip comparisons between an experimental group, as defined above, and naïive (untrained) animals will yield CPGs, along with CMGs.

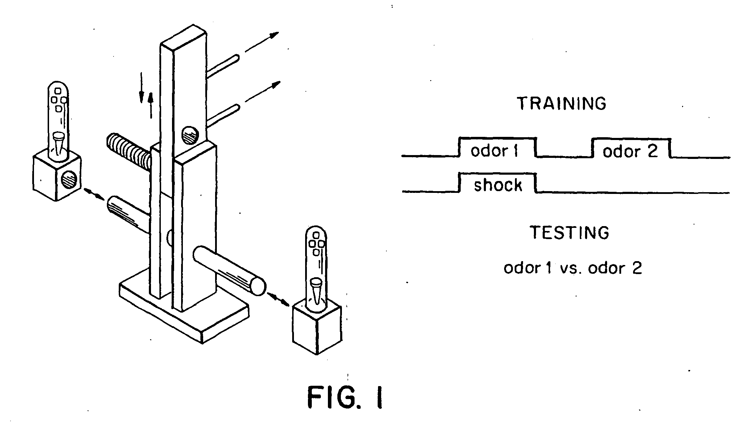

Behavior-genetic studies in Drosophila have established a pair of training protocols with differential effects on memory formation after a Pavlovian odor-shock learning paradigm. Ten training sessions "massed" together (i.e., with no rest interval between sessions) yields maximal learning (acquisition) and transcription-independent memories (not protein synthesis-dependent) (early memories, short-term memory). In contrast, ten training sessions "spaced" (i.e., with a 15-minute rest interval between sessions) yields equivalent levels of learning and transcription-independent memories (early memories), as well as maximal levels of transcription-dependent memory (including protein synthesis-dependent long-term memory (LTM)). LTM requires spaced training; even 48 massed training sessions fails to induce LTM (Tully et al., Cell, 79:35-47 (1994)). Protein synthesis-dependent LTM induced by spaced training is blocked completely via overexpression of CREB repressor (Figure 1) (Yin et al., Cell, 79:49-58 (1994)). The resulting memory curve after spaced training, where protein synthesis- and CREB-dependent LTM is blocked, is similar to that produced by massed training in normal flies. In contrast, overexpression of CREB activator induces LTM with less training (one training session) or with massed training (Figure 2) (Yin et al., Cell, 81:107-115 (1995)). Hence, the induction of LTM is both protein synthesis- and CREB-dependent. These results demonstrate that the only functional difference between spaced and massed training protocols is the appearance of transcription-dependent memory after the former.

This observation forms the basis of a differential screen to identify additional "downstream" genes that are transcriptionally regulated during transcription-dependent memory formation. DNA probes were synthesized using RNA extracted from the heads of spaced- or massed-trained flies according to methods generally known in the art (see, e.g., Sambrook et al., Eds., Molecular Cloning: A Laboratory Manual, 2nd edition, Cold Spring Harbor University Press, New York (1989); and Ausubel et al., Eds., Current Protocols In Molecular Biology, John Wiley & Sons, New York (1997)). RNA was extracted from fly heads as described previously (see, e.g., Drain et al., Neuron, 6:71-82 (1991)). Spaced- and massed-training of flies were conducted as described previously (see, e.g., Tully et al., Cell, 79:35-47 (1994); and Tully and Quinn, J. Comp. Physiol., 157:263-277 (1985)). Complementary DNA (cDNA) probe was synthesized from the extracted RNA according to methods generally known in the art (see, e.g., Sambrook et al., Eds., Molecular Cloning: A Laboratory Manual, 2nd edition, Cold Spring Harbor University Press, New York (1989); and Ausubel et al., Eds., Current Protocols In Molecular Biology, John Wiley & Sons, New York (1997)). The complex cDNA probe mixture then was hybridized onto microarray chips containing DNA sequences (target DNA sequences) of 1542 *Drosophila* genes (Affymetrix, Inc., Santa Clara, CA; see also, e.g., U.S. Patent No. 5,445,934; and Ramsay, Nature Biotechnology, 16: 40-44 (1998)). In a particular embodiment, the DNA probes are labeled with a detectable marker (e.g., fluorescent marker). The signal from hybridized DNA probes was amplified and detected according to methods generally known in the art (see, e.g., Sambrook et al., Eds., Molecular Cloning: A Laboratory Manual, 2nd edition, Cold Spring Harbor University Press, New York (1989); and Ausubel et al., Eds., Current Protocols In Molecular Biology, John Wiley & Sons, New York (1997)). In a particular embodiment, hybridization was detected using a fluorescence assay. A statistical comparison (DNA chip comparison) was made (performed) by comparing the signal detected between spaced- and massed- trained groups.

A sample size and signal transformation algorithm has been determined that improves the statistical reliability to detect small differences in transcript levels between spaced- and massed-trained groups. In a preferred embodiment, it has been determined that a sample size of 10 chips per treatment group for each treatment protocol (i.e., for each gene, 10 chips for spaced-trained group and 10 chips for massed-trained group) improves the statistical reliability to detect small differences in transcript levels between spaced- and massed-trained groups. In a preferred embodiment, statistical comparison (DNA chip comparison) is made using the following signal transformation algorithm:

> 1. Determine the "average difference" between signal detected for a set of primer pairs for specific gene. The average difference between perfect matched (PM) and mismatched (MM) signals is determined by Affymetrix design software analysis (Affymetrix, Inc., Santa Clara, CA).
>
> 2. Box-Cox Transformation:
>
>> Any average difference value below 10 is eliminated. The remaining average difference values for each gene on each chip then are normalized by the overall average difference (across all genes) for that entire chip.
>
> Grand Mean = Overall Avg. Diff. for all chips and all genes/chip

$$\text{Normalization Factor (for Chip X)} = \frac{\text{Overall Avg. Diff. for Chip X}}{\text{Grand Mean}}$$

$$\text{Norm (Avg. Diff.)} = (\text{Norm. Factor for Chip X}) \times (\text{Gene Y on Chip X})$$

$$\text{Transformed Avg. Diff.} = \{\ln [\text{norm(Avg. Diff.)}]\} \times 2720.75$$

> 3. Determine the mean and standard error. Compare mean for spaced- and massed-trained flies for each Gene Y using standard t-tests (alpha = 0.05). If $p \leq 0.05$, then the mean signal transformation for a given gene in spaced-trained flies is considered to be statistically different from the mean signal transformation for that gene in massed-trained flies.

Alternatively, statistical comparison (DNA chip comparison) can be made using the following signal transformation algorithm:

> 1. Determine the "average difference" between signal detected for a set of primer pairs for specific gene. The average difference between perfect matched (PM) and mismatched (MM) signals is determined by Affymetrix design software analysis (Affymetrix, Inc., Santa Clara, CA).
>
> 2. Box-Cox Transformation:
>
>> Any negative average difference for a given gene is zeroed. The "average difference zeroed" (Avg. Diff. 0) for each gene on each chip is normalized by the overall "average difference" for that entire chip.
>
>> a)

$$\text{Avg. Diff. } 0 = \text{Avg. Diff., if Avg. Diff.} \geq 0,$$
$$= 0, \text{ if Avg. Diff.} < 0$$

b) Normalization:

$$\text{Grand Mean} = \text{Overall Avg. Diff. for all chips and all genes/chip}$$

$$\text{Normalization Factor (for Chip X)} = \frac{\text{Overall Avg. Diff. for Chip X}}{\text{Grand Mean}}$$

Norm (Avg. Diff. 0) = Normalization Factor (for Chip X) $\times$ (Gene Y on Chip X) (for each gene)

c)

$$\text{Transformed Avg. Diff.} = \{\ln [\text{norm(Avg. Diff. 0)}]\} \times 2720.75136$$

3. Determine the mean and standard error. Compare spaced mean versus massed mean with a t-test comparison. If $p \leq 0.05$, then the mean signal transformation for a given gene in spaced-trained flies is considered to be statistically different from the mean signal transformation for that gene.in massed-trained flies.

Other signal transformation algorithms can be used in performing a statistical comparison (DNA chip comparison) of signal detected between spaced- and massed-trained groups.

With these proprietary approaches, 1542 statistical spaced versus massed training comparisons (Student t-tests) have been determined for Drosophila. Candidate memory genes (CMGs) (not including transposable elements or mitochondrial genes), which are transcriptionally regulated during transcription-dependent memory formation, were identified from the DNA chip comparisons (statistical comparisons) between spaced- and massed-trained groups. Transcripts that are differentially regulated represent those involved specifically in "associative" LTM induced by a Pavlovian odor-shock learning paradigm.

CMGs from Drosophila determined from statistical analysis using the signal transformation algorithm in which an average difference value below 10 for a given gene is deleted, from the spaced versus.massed comparison (24-hour memory), are presented in Table I. CMGs from Drosophila determined from statistical analysis using the signal transformation algorithm in which a negative average difference value for a given gene is zeroed, from the spaced versus massed comparison (24-hour memory), are presented in Table 2.

| TABLE 1: Statistical Candidate Memory Genes, derived from statistical analysis (delete avg. diff. values < 10), from the spaced versus massed comparison (24-hour memory). |
|---|
| hikaru genki type1 product <br> inositol 1,4,5-trisphosphate receptor <br> mitochondrial cytochrome c oxidase subunits, ATPase6, 7 tRNAs <br> mitochondrial cytochrome c oxidase subunits, ATPase6, 7 tRNAs <br> mitochondrial cytochrome c oxidase subunits, ATPase6, 7 tRNAs <br> C/EBP gene <br> disabled <br> molybdenum cofactor (cin) <br> dif <br> syntaxin1A (syx-1A) <br> fsh membrane protein, 7.6 kb mRNA |

(continued)

| TABLE 1: Statistical Candidate Memory Genes, derived from statistical analysis (delete avg. diff. values < 10), from the spaced versus massed comparison (24-hour memory). |
| --- |
| defective chorion fc 177 (dec-1) gene |
| mitochondrial DNA with 12 tRNAs and 7 genes. |
| proteasome (PROSA-28.1.1.) |
| cysteine-string protein 32 (csp32) |
| FTZ-F1 RNA |
| Mov34 protein mRNA |
| transcription factor TFIID 230 kda subunitnt |
| croc/FD1 = crocodile |
| CS-5 pheromone-binding protein homolog OS-E mRNA |
| mago-nashi protein (mgn) gene |
| transcription initiation factor TFIID 28 kDa subunit mRNA |
| Canton S RNA binding protein La/SS-B (DLa/SS-B) mRNA |
| angiotensin converting enzyme precursor (Ance) mRNA |
| commissureless (comm) mRNA |
| nuclear hormone receptor superfamily member DHR78 (DHR78) mRNA |
| larval serum protein 1 beta subunit (Lsp-1b) gene |
| cut locus mRNA for homeodomain-containing protein |
| DRI class II gene for type I regulatory subunit of cAMP-dependent kinase |
| PO gene |
| PP1 13C gene for protein phosphatase 1 13C |
| mRNA for 51 kDa protein |
| genes mst 355a and 355b for male accessory gland secretory protein |
| PP-Y mRNA for protein phosphatase Y (EC 3.1.3.) |
| anon-66Da, Minute(3)66D and anon-66Db genes |
| mRNA for 5HT-dro2A receptor (serotonin receptor) |
| eye color protein (garnet) mRNA |
| sparkling protein (spa) mRNA |
| kinesin-related protein (costal-2) mRNA |
| Fuzzy (fuzzy) mRNA |
| chitinase (CHT4) gene |
| mRNA for rab11 |
| neu=neuralized mRNA |
| anachronism Genomic/mRNA |
| TART-B1 transposon putative single-stranded nucleic acid bind |
| sodium channel protein (para) gene, exons 9,10,11,12 |
| mod2.2 (mod(mdg4)) mRNA |
| tyrosine kinase mRNA |
| leucine-rich repeat/Ig transmembrane protein KEK1 precursor |
| gliolectin mRNA |
| Deformed epidermal autoregulatory factor-1 (Deaf1) mRNA |
| Lozenge (lz) mRNA |
| hook (hook) mRNA |
| SH2/SH3 adaptor protein (Dock) mRNA |
| retinoid- and fatty acid-binding glycoprotein mRNAblown fuse protein (blow) mRNA |
| GCR 101 mRNA |
| mRNA for metabotropic glutamate receptor |
| mRNA for ladybird late homeodomain transcription factor |

(continued)

| TABLE 1: Statistical Candidate Memory Genes, derived from statistical analysis (delete avg. diff. values < 10), from the spaced versus massed comparison (24-hour memory). |
| --- |
| mRNA for putative mitochondrial protein, partial |
| colt gene |
| mRNA for nuclear protein SA |
| hikaru genki type gene |
| tyrosine kinase hopscotch gene |
| syntaxin-1A (syx-1A) gene |
| proteasome (PROSA-28.1.1.) gene |
| P-glycoprotein (MdR 49) gene |
| Mov34 protein gene |
| POU domain protein (pdm-1) gene |
| Drosophilia melanogaster epidermal growth factor-like protein (spitz) gene |
| (clone 10B-1) germ cell-less protein (gc11) gene |
| mago-nashi protein (mgn) gene |
| serotonin transporter gene |
| transcription initiation factor TFIID 28 kDa subunit gene |
| ribonucleoside-diphosphate reductase large subunit gene |
| nudel (ndl) gene |
| bithorax complex (BX-C) gene cluster |
| commissureless (comm) gene |
| DNA polymerase gamma gene |

| TABLE 2: Statistical Candidate Memory Genes from Drosophila, derived from statistical analysis (negative avg. diff. value is zeroed), from the spaced versus massed comparison (24-hour memory). |
| --- |
| larval serum protein 1 beta subunit (Lsp-1b) gene |
| hdl cuticle gene cluster |
| alpha-methyldopa hypersensitive gene 1(2)amd gene |
| Shaker ShB gene |
| DNA-binding protein Elf1 gene |
| chorion protein s16 gene |
| Adh and Adh-dup genes |
| E2F gene |
| 51 kDa protein gene |
| caupolican homeoprotein gene |
| 5HT-dro2A receptor gene |
| odorant binding protein LUSH (lush) gene |
| eye color protein (garnet) gene |
| adenylyl cyclase isoform DAC9 gene |
| kinesin-related protein (costal-2) gene |
| chitinase (CHT1) gene |
| canoe gene |
| rab11 gene |
| imitation-SWI protein (ISWI) gene |
| receptor guanylyl cyclase (DGC1) gene |
| tumor supressor (warts) gene |
| kinesin-like protein (KLP4) gene |
| myosin-IA gene |
| cytoplasmic basic protein (deltex) gene |

(continued)

| TABLE 2: Statistical Candidate Memory Genes from Drosophila, derived from statistical analysis (negative avg. diff. value is zeroed), from the spaced versus massed comparison (24-hour memory). |
| --- |
| Ca/calmodulin-dependent nitric oxide synthase (NOS) gene |
| sodium channel protein (para) gene |
| CKII alpha subunit interactor 1 (CKIIalpha-I1) gene |
| leucine-rich repeat/Ig transmembrane protein KEK1 precursor (kek1) gene |
| geranylgeranyl transferase beta-subunit type I (beta GGT-I) gene |
| hook (hook) gene |
| SH2/SH3 adaptor protein (Dock) gene |
| RNA-binding protein lark (lark) gene |
| retinoid-and fatty acid-binding glycoportein gene |
| Dreg-2 protein gene |
| transcription factor dMax gene |
| non-histone chromosomal protein Prod (prod) gene |
| blown fuse protein (blow) gene |
| orb gene |
| angel gene |
| ladybird late homeodomain transcription factor gene |

The statistical procedures described above only suggest "statistical candidates." A fundamental aspect of the statistical methods employed (as well as other such methods) is that "false positive" and "false negative" candidates are obtained along with the "true positives." Hence, an independent method of detecting experience-dependent changes in gene transcription must be applied to the "statistical candidates." Such independent methods include Northern blot analysis, quantitative polymerase chain reaction (QPCR) and RNase protection assays, and can be used to confirm the statistical candidates identified. The quantitative analyses of these data also are subject to false positive and false negative results.

Minor changes in the statistical methods herein can yield a different set of "statistical candidates". Often times, more than one type of data transformation is sufficient to yield a normalized distribution of difference scores. Each data transformation used, however, will yield a different set of statistical candidates. All methods of signal detection also must resolve "baseline values", which are too low for accurate detection. Setting such values to "zero" is one way to deal with this difficulty. Another way is to eliminate such values from the data set (e.g., eliminate values of less than 10, for example).

Chip data provide confirmatory information, gene-by-gene, as to which transcripts are involved with memory. Chip data also provide exact coordinated transcriptional response to different stimuli across all gene transcripts. In particular, chip data provide information as to the coordinated effect a gene transcript has on memory.

Most genes in Drosophila have been shown to have mammalian homologs, and such is the case for most Drosophila genes involved in memory formation (Dubnau and Tully, Ann. Rev. Neurosci., 21:407-444 (1998)). With the growing knowledge that mammalian homologs can be functionally substituted in Drosophila for its fly homolog, the present discovery directly implicates the corresponding mammalian homologs.

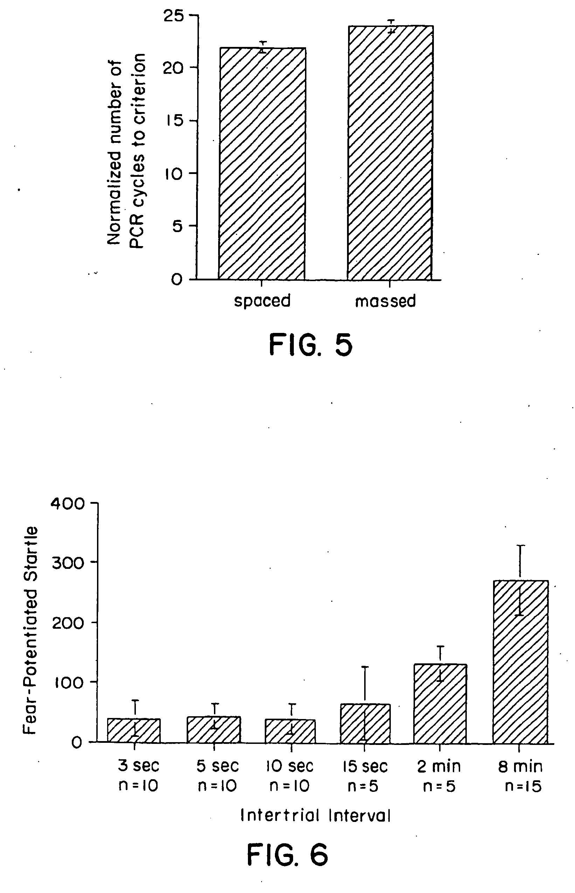

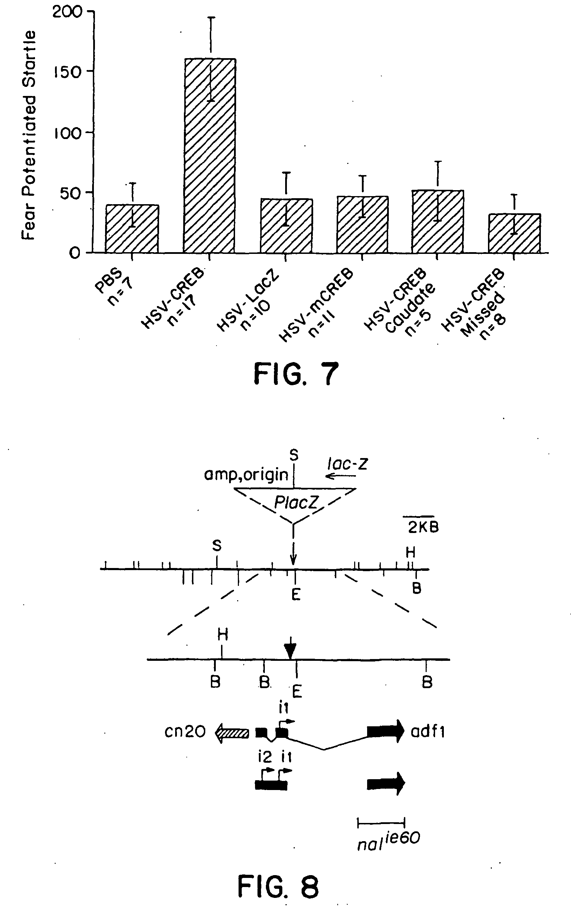

The differential effects on long-lasting memory produced by spaced versus massed training is a phenomenon widely observed in the animal kingdom. In particular, a spaced-massed differential effect on long-lasting memory recently has been established for the conditioned fear-potentiated startle effect in rats (a mammalian model system). In the fear-potentiated startle paradigm, memory is inferred from an increase in startle amplitude in the presence of a conditioned stimulus (CS) that has been previously paired with footshock. Massed training in rats (4-CS-shock pairings with a 10-second intertrial interval) produces essentially no transcription-dependent memory whereas spaced training (4 pairings with an 8-minute intertrial interval) produces significant transcription-dependent memory (Figure 6) (Josselyn et al., Society for Neurosci., 24: 926, Abstract 365.10 (1998)). Moreover, overexpression of CREB activator, delivered to the amygdala via viral vector technology, enhances memory from massed training in a manner directly analogous to that observed in Drosophila (Figure 7) (Josselyn et al., Society for Neurosci., 24: 926, Abstract 365.10 (1998)). These data demonstrate a CREB-dependent spaced-massed differential with which to identify mammalian CMGs.

Hence, these specific training protocols are expected to yield CMGs in animals, such as mammals, similar to the CMGs identified in Drosophila. The term "animal", as used herein, includes mammals, as well as other animals, vertebrate and invertebrate (e.g., birds, fish, reptiles, insects (e.g., Drosophila species), Aplysia). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and ruminents (e.g., cows, pigs, horses).

To identify the CMGs in non-human animals (particularly non-human mammals, other vertebrates and invertebrates), DNA probes are synthesized using RNA extracted from brain tissues of spaced- or massed-trained non-human animals according to methods generally known in the art (see, e.g., Sambrook et al., Eds., Molecular Cloning: A Laboratory Manual, 2nd edition, Cold Spring Harbor University Press, New York (1989); and Ausubel et al., Eds., Current Protocols In Molecular Biology, John Wiley & Sons, New York (1997)). These probes can be labeled with a detectable marker. In a particular embodiment, DNA probes are synthesized using RNA extracted from the amygdala of spaced- or massed-trained animals and, if required, labeled with a detectable marker. A variety of detectable markers and labeling methods are known in the art, including fluorescent, chemiluminescent, biotin, radioactive, enzymatically detected and immunologically detected markers (see, e.g., Sambrook et al., Eds., Molecular Clowning: A Laboratory Manual, 2nd edition, Cold Spring Harbor University Press, New York (1989); and Ausubel et al., Eds., Current Protocols In Molecular Biology, John Wiley & Sons, New York (1997)). RNA is extracted from brain tissues, such as the amygdala, according to methods available in the art. Spaced- and massed-training of animals are conducted using methods generally known in the art (see, e.g., Josselyn et al., Society for Neurosci., 24: 926, Abstract 365.10 (1998); Cassella and Davis, Physiol. Behav., 36:377-383 (1986); Guzowski et al., Proc. Natl. Acad Sci. USA, 94:2693-2698 (1997); Lamprecht et al., J. Neuroscience, 17(21):6443-6450 (1997); Bourtchuladze et al., Cell, 79:59-68 (1994); and Kogan et al, Curr. Biol., 7:1-11 (1996)). This complex probe mixture then is hybridized onto microarray chips containing DNA sequences (target DNA sequences) of genes of the genome of the animals (see, e.g., U.S. Patent No. 5,445,934; and Ramsay, Nature Biotechnology, 16: 40-44 (1998)). The signal from hybridized DNA probes is amplified and detected according to methods generally known in the art (see, e.g., Sambrook et al., Eds., Molecular Cloning: A Laboratory Manual, 2nd edition, Cold Spring Harbor University Press, New York (1989); and Ausubel et al., Eds., Current Protocols In Molecular Biology, John Wiley & Sons, New York (1997)). For example, hybridization (the signal from the hybridized probe) can be detected using fluorescence assays or mass spectrometry. Methods using optical fibers, diode array detection, chemiluminescence/luminescence, latex bead agglutination, direct electrical charge change detection (CCD) and piezoelectric readout can also be used. The signal transformation algorithm described above is used to calculate gene expression levels between spaced- and massed-trained groups. A statistically significant difference in transcript level between spaced- and massed-trained groups for a specific gene identifies a candidate memory gene. Statistical comparison (DNA chip comparison) between spaced- and massed- trained groups can be made using the signal transformation algorithms described above.

In addition to statistical comparisons between spaced- and massed-trained groups, CMGs can be identified from DNA chip comparisons (statistical comparisons) between animals trained using other pairs of experimental protocols. The experimental group is trained under conditions sufficient to induce transcription-dependent memory and the control group is trained under conditions that are not sufficient to induce transcription-dependent memory. The significant difference between any two experimental protocols is in the induction of transcription-dependent memory. The significant difference between any two experimental protocols to be compared is the induction of transcription-dependent memory in the experimental group and the absence of transcription-dependent memory in the control group.

Pairs of experimental protocols that primarily differ in the induction of transcription-dependent memory are known the art. For example, a pair of experimental protocols that primarily differ in the induction of transcription-dependent memory can consist of a spaced training protocol and a massed training protocol. In this embodiment, training an animal using a spaced training protocol is sufficient to induce transcription-dependent memory in the animal. Training an animal using a massed training protocol is not sufficient to induce transcription-dependent memory. As another example, a pair of experimental protocols that primarily differ in the induction of transcription-dependent memory can consist of training a normal (wildtype) animal using a shuttle-box avoidance protocol (particularly a one-trial shuttle-box avoidance protocol) and training an animal in which the fornix is surgically lesioned using the shuttle-box avoidance protocol (Taubenfeld et al., Nat. Neurosci., 2(4):309-310 (1999)). In this embodiment, transcription-dependent memory is induced in the normal (wildtype) animal. Transcription-dependent memory is not induced in the animal in which the fornix is surgically lesioned. As a further example, a pair of experimental protocols that primarily differ in the induction of transcription-dependent memory can consist of training an animal using a contextual fear conditioning protocol (particularly a one-trial contextual fear conditioning protocol) and training an animal habituated to the training chamber before contextual fear conditioning using the contextual fear conditioning protocol (Imprey et al., Nat. Neurosci., 1(7):595-601 (1998)). In this embodiment, transcription-dependent memory is induced in the animal that had not been habituated to the training chamber prior to contextual fear conditioning. Transcription-dependent memory is not induced in the animal habituated to the training chamber before contextual fear conditioning. Other pairs of experimental protocols can readily be identified by those skilled in the art.

DNA probes are synthesized using RNA extracted from the brain tissues, such as the amygdala and hippocampus, of animals trained using pairs of experimental protocols, as described herein, and, if required, labeled with a detectable marker. The DNA probe mixtures then are hybridized onto microarray chips containing DNA sequences (target DNA sequences) of genes of the genome of the animals. A statistical comparison is made by comparing the DNA chip data between the two experimental protocols using the signal transformation algorithms described above.

The statistical procedures herein can be used to detect differences in transcript levels between trained and untrained (naïve) groups. Accordingly, candidate plasticity genes (CPGs) can be identified from DNA chip comparisons (statistical comparisons) between trained versus untrained (naïve) animals. Transcripts that are differentially regulated in this class will include the CMGS, along with any other genes that are transcriptionally responsive in a "nonassociative" manner to the general training conditions (e.g., presentations of odors, electroshock or any other experiential aspects of the training protocol). Some nonspecific transcriptional responses occur simply when an animal is placed in a novel environment or when the animal is exposed to a stimulus alone or unpaired in time. These transcriptional changes may result from general (nonspecific) increases in neuronal activity or reflect other forms of learning/memory formation that are not related to the general training conditions.

CPGs from Drosophila determined from statistical analysis using the signal transformation algorithm in which an average difference value below 10 for a given gene is deleted, from the spaced versus naive comparison (24-hour memory), are presented in Table 3. CPGs from Drosophila determined from statistical analysis using the signal transformation algorithm in which a negative average difference value for a given gene is zeroed, from the spaced versus naive comparison (24-hour memory), are presented in Table 4.

| TABLE 3: Statistical Candidate Plasticity Genes, derived from statistical analysis (delete avg. diff. values < 10), from the spaced versus naive comparison (24-hour memory). |
|---|
| ribosomal protein S6 (rps6) |
| hu-li tai shao (hts) mRNA |
| ribosomal protein S3/AP endonuclease DNA repair protein mRNA |
| gurken gene |
| atonal protein mRNA |
| cytoplasmic dynein intermediate chain (Cdic) gene |
| glyceraldehyde-3-phosphate dehydrogenase-1 gene |
| glyceraldehyde-3-phosphate dehydrogenase-2 gene |
| 50 kDa protein F1 gene |
| Dmras85D gene |
| serine protease (SER1 and SER2) genes |
| myosin light chain 2 (MLC2) mRNA |
| carboxylesterase 6 and P (Est-6 and Est-P) genes |
| annexin IX mRNA |
| proteasome (PROSA-28.1.1.) mRNA |
| laminin B2 gene |
| octopamine receptor mRNA |
| A2 component of diphenol oxidase (Dox-A2) gene |
| homolog of RAD6 (DHR6) mRNA |
| Mov34 protein mRNA |
| glu-prolyl tRNA aminoacyl synthetase mRNA |
| G protein-coupled receptor kinase (GPRK-1) mRNA |
| optomotor-blind mRNA |
| profilin (chickadee) mRNA |
| transcription factor IIB (TFIIB) mRNA |
| glutathione S-transferase-related protein mRNA |
| trypsin-alpha, -beta and -epsilon genes |
| trypsin-alpha, -beta and -epsilon genes |
| catalase gene |
| proteasome subunit (1(3)73Ai) gene |
| Canton S pheromone-binding protein-related protein PBPRP-2 mRNA |
| transcription initiation factor TFIID 28 kDa subunit mRNA |
| glutathione-dependent formaldehyde dehydrogenase gene |
| cofilin/actin depolymerizing factor homolog mRNA |
| N-ethylmaleimide-sensitive fusion protein mRNA |

(continued)

| TABLE 3: Statistical Candidate Plasticity Genes, derived from statistical analysis (delete avg. diff. values < 10), from the spaced versus naive comparison (24-hour memory). |
| --- |
| ribosomal protein DL11 mRNA |
| rfc40 protein, Rop protein (Rop), and small GTP binding protein |
| PROS-Dm25g gene for proteasome |
| DmTnC 41C mRNA for troponin-C |
| WM6 mRNA |
| mRNA for 40S ribosomal protein S12 |
| mRNA for mitochondrial ATPase synthase |
| genes mst 355a and 355b for male accessory gland secretory protein |
| Pgk gene for phosphoglycerate kinase |
| D.melanogaster ribosomal protein 15a (40S subunit). |
| eye color protein (garnet) mRNA |
| cysteine proteinase-1 (CP1) gene |
| calcium-binding protein (SCP1) mRNA |
| cytochrome P450 (CYP4D2) gene |
| alpha NAC (oxen) gene, complete cds; and G76C pseudogene |
| alpha NAC (oxen) gene, complete cds; and G76C pseudogene |
| kinesin-related protein (costal-2) mRNA |
| transcriptional co-repressor SIN3A (Sin3A) mRNA |
| chitinase (CHT2) gene |
| mRNA for rab-related protein 4. |
| mRNA for still life type 1 |
| cell adhesion molecule encoding (nrm) gene |
| phosphoglycero mutase (Pglym78) gene |
| receptor guanylyl cyclase (DGC1) mRNA |
| (W-IR1 mutation) I factor DNA |
| (W-IR1 mutation) I factor DNA |
| Fw repetitive element putative reverse transcriptase |
| Fw repetitive element putative reverse transcriptase |
| RNA polymerase II second largest subunit upstream (DmRP 140) |
| ecdysone-inducible membrane (IMP-L1) gene |
| mdglhet, integrase {MDG1 retrotransposon} |
| Dacp-1=cuticle protein |
| glutamate decarboxylase mRNA |
| HeT-A element 23Zn-1. |
| (zeste-white 4) mRNA |
| Dachshund (dachshund) mRNA |
| Hk protein mRNA |
| soluble guanylyl cyclase beta subunit (dgcb1) |
| cytochrome P450 (Cyp4g1) mRNA |
| alpha esterase (aE10) gene |
| vacuolar ATPase subunit A (vha68-2) gene |
| fatty acid desaturase mRNA |
| wunen gene |
| Rga and Atu genes |
| kinesin-73 mRNA |
| MCM5 homolog (DmMCMS) mRNA |
| kinesin like protein 67a mRNA |
| sperm-specific protein component (dj) mRNA |

(continued)

| TABLE 3: Statistical Candidate Plasticity Genes, derived from statistical analysis (delete avg. diff. values < 10), from the spaced versus naive comparison (24-hour memory). |
|---|
| DNA sequence (isolate CBS) for 18S rRNA (3'end), 5.8S rRNA and 28S rRNA |
| nmr mRNA for DNMDAR-I |
| mRNA for angiotensin-converting enzyme-like protein |
| mRNA for histone H4 |
| mRNA for ladybird late homeodomain transcription factor |
| colt gene |
| mRNA for ATP synthase subunit gamma |
| mRNA for 3-hydroxyacyl-CoA-dehydrogenase type II |

| TABLE 4: Statistical Candidate Plasticity Genes, derived from statistical analysis (negative avg. diff. value is zeroed), from the spaced versus massed comparison (24-hour memory). |
|---|
| daughterless protein (da) |
| steroid receptor (FTZ-F1B) |
| POU domain protein (pdm-1) |
| bithorax complex (BX-C) |
| nuclear hormone receptor superfamily(DHR96) |
| transcriptional co-repressor SIN3A (Sin3A) |
| mRNA for histone H4 |
| mRNA for ladybird late homeodomain |
| RAD6 (DHR6) |
| putative serine protease (easter) |
| serine protease (SER1 and SER2) genes |
| proteasome (PROSA-28.1.1.) |
| Mov34 protein |
| trypsin-alpha, -beta and -epsilon |
| trypsin-alpha, -beta and -epsilon genes |
| proteasome subunit (1(3)73Ai) gene |
| 20S proteasome alpha subunit PSMA5 gene |
| snake locus mRNA for serine protease |
| D.melanogaster PROS-Dm25g gene for proteasome |
| male accessory gland secretory protein (serpin) |
| serine protease SER4 precursor (Ser4) |
| cysteine proteinase-1 (CP1) gene |
| clone 6 serine protease mRNA |
| Drosophila melanogaster dishevelled mRNA, complete cds. |
| Dmras85D gene, exon 3 |
| G protein-coupled receptor kinase (GPRK-1) mRNA |
| rfc40 protein, Rop protein (Rop), and small GTP binding protein |
| tyrosine kinase, partial sequence |
| mRNA for rab-related protein 4 |
| mRNA for rab-related protein 3 |
| GDP dissociation inhibitor homologue (dGDI) mRNA |
| (zeste-white 4) mRNA |
| phosphoinositide 3-kinase, Dp 110 |
| phosphatase 2A catalytic subunit |
| fasciclin III mRNA |

(continued)

| TABLE 4: Statistical Candidate Plasticity Genes, derived from statistical analysis (negative avg. diff. value is zeroed), from the spaced versus massed comparison (24-hour memory). |
| --- |
| annexin IX mRNA |
| alpha-methyldopa hypersensitive gene 1(2)amd |
| GS2 mRNA for glutamine synthase |
| GS1 mRNA for glutamine synthase |
| mRNA for dopamine receptor |
| eye color protein (garnet) mRNA, clathrin like |
| cell adhesion molecule encoding (nrm) gene |
| Nrv 2.2 neuron surface antigen 2 (Nrv2) mRNA |
| R vacuolar ATPase subunit A (vha68-2) gene |
| mRNA for DNMDAR-I |
| AcTr66B gene for actin-related protein |
| DmTnC 41C mRNA for troponin-C |
| kinesin-related protein (costal-2) mRNA |
| microtubule associated protein (asp) mRNA |
| cytoplasmic dynein intermediate chain (Cdic) gene |

Transcription-dependent memory can be induced using specific experimental conditions. In one embodiment, transcription-dependent memory is induced in a non-human animal using a spaced training protocol for the fear-potentiated startle response. In a second embodiment, transcription-dependent memory is induced in a non-human animal using a shuttle-box avoidance protocol. In a third embodiment, transcription-dependent memory is induced in a non-human animal using a contextual fear conditioning protocol.

The present invention will now be illustrated by the following examples, which are not to be considered limiting in any way.

EXAMPLES

EXAMPLE 1 Isolation of *nalyot*[P1].

An X-linked *PlacW* transposon (Bier et al., Science, 240(4854):913-916 (1988)) was mobilized to generate 2,182 transposant strains with independent insertions on the second and third chromosomes (cf. Boynton and Tully, Genetics, 131:655-672 (1992); Dura et al., J. Neurogent., 9:1-14 (1993)). Three-hour memory after a single training session of Pavlovian olfactory learning was quantified with one performance index (PI) for each of these transposant strains. N = 4 PIs then were generated for those strains that scored 70% or less of a wild-type parental strains [W[1118] (CS10)]. At this stage of the screen, 93 mutant strains yielded mean three-hour memory scores 70% wild-type or less. Each of these candidate mutant strains then was outcrossed for five generations to the parental strain to equilibrate their (heterogeneous) genetic backgrounds. When three-hour memory again was quantified (N = 4 PIs) in these outcrossed strains, only eight of the 93 candidate mutants still yielded mean scores < 70% wildtype. Finally, "task relevant" sensorimotor tasks were assayed in these eight mutant strains. All eight showed normal shock reactivity; four, $G_B335$ and $E_J51$, $E_J220$ and $E_S152$, showed significantly reduced olfactory acuity (Boynton and Tully, Genetics, 131:655-672 (1992); Dura et al., J. Neurogent., 9:1-14 (1993). [$G_B335$, now named *dare,* has been studied further and shows preferential expression in antenna (Freeman et al., Development, 126:4591-4602 (1999)]. The remaining four mutant strains displayed normal sensorimotor responses and were named *latheo* (Boynton and Tully, Genetics, 131:655-672 (1992); Pinto et al., Neuron, 23:45-54 (1999); Rohrbough et al., Neuron, 23:55-70 (1999), *linotte* (Dura et al., J. Neurogent., 9:1-14 (1993); Bolwig et al., Neuron, 15:829-842 (1995); Simon et al., Mech. Dev., 76:42-55 (1998), *golovan* and *nalyot.*

EXAMPLE 2 Cloning and Characterization of *nalyot* Genomic Region.

The *PlacW transposon* includes a unique SacII restriction site followed by the bacterial origin of replication and ampicillin resistance gene (Figure 8). Digestion of *nalyot* (*nal*) genomic DNA with SacII ligation under dilute conditions and bacterial transformation allowed plasmid rescue of a 9.4 kb SacII restriction fragment along with flanking DNA from the genomic region. Chromosome in situs and southern blotting experiments verified that this fragment co-mapped to the P-insertion site. The radiolabeled rescue fragments were used to screen one million plaques of a lambda-DashII *Drosophila* Can-S genomic library (Stratagene). Isolation, subcloning and restriction analysis of 10 independent genomic clones led to the construction of a 35 kb map spanning the genomic region around the P-element insertion site (Figure 8).

Intron/exon maps of the *adf1* and *cn20* transcription units are shown in Figure 8. The *nal*[P1] element (arrow) is inserted within an intron of the *adf1* transcription unit, 147 bp downstream of the splice donor site. The *adf1* gene encodes a transcription factor distantly related to the myb family (England et al., Proc. Natl. Acad. Sci. USA, 89:683-687 (1992)) and is alternatively spliced into (at least) two mRNAs. i1 and i2 correspond to two potential translation start sites. Two additional introns of about 3.5 kb and 59 bp appear to be spliced constitutively and separate the remainder of the adf1 transcription unit into 274 bp and 1,013 bp exons. The *cn20* gene is novel and produces a single, unspliced transcript that can encode a 395 amino acid protein. The extent of the genomic deletion in *nal*[le60] is indicated. Restriction sites: B, BamHI; E, EcoRI; H, HindIII; S, SacII.

EXAMPLE 3 Northern Blot Analysis of *adf1* and *cn20* In Mutant *nal*[P1] and Wildtype Flies and cDNA Isolation.

Total RNA from whole adult flies, adult heads or adult bodies was isolated with the TriZOL reagent (BRL). The poly(A) fraction was subsequently purified with oligo(dT) cellulose (Collaborative Research) or magnetized oligo(dT) beads (Dynal). Purified poly(A) RNA was fractionated by formaldehyde-agarose gel electrophoresis and transferred to a ZetaProbe nylon membrane (BioRad) in 10×SSC. The RNA on the dried membrane was fixed by UV-crosslinking at 2,500 ujoules (Stratalinker). For initial identification of transcript classes, membrane strips were probed overnight with radiolabeled genomic DNA fragments in high stringency Church and Gilbert Buffer, washed extensively and exposed to Kodak BioMax film.

Selected probes were hybridized to two *Drosophila* adult head cDNA libraries, a lambda gt11 bacteriophage adult head library (Salvaterra) and a pJG4-5 plasmid library (Roshbash). From these two libraries, a total of eleven clones, corresponding to two independent transcription units, were isolated and evaluated by restriction analysis. Ten clones corresponded to the *adf1* transcription unit. Restriction-mapping and sequence analysis of a subset of these revealed a common 3' end processing site and heterogeneity at the 5' end. The 5' heterogeneity reflected the partial splicing of intron 1 (114 bp) and, perhaps, incomplete first strand synthesis. One clone, cn20, corresponded to an independent, neighboring transcription unit.

To quantify the effect of the P-insertion on *adf1* and *cn20* RNA levels, Northern blots derived from *nol*[P1] and wildtype heads or bodies were analyzed as above with radiolabeled probes corresponding to *adf1, cn20* and a control RNA *rp49.*

Relative to control levels of *rp49* RNA, *cn20* mRNA expression levels in both heads and bodies were similar in wildtype and mutant flies. In contrast, *adf1* mRNA expression levels were reduced by at least two-fold in mutant heads and bodies.

EXAMPLE 4 Antibody Production.

The entire *ADFI* open reading frame was inserted into the pET30(a) expression vector (Novagen) as a C-terminal fusion. Robust IPTG induction of ADF1 fusion protein was obtained in transformed BL21 bacteria. The majority of induced protein was in the insoluble, inclusion body fraction and this fraction (isolated 3 hours after induction) was enriched nearly 85% for the ADF1 fusion protein. This fraction was washed extensively with PBS and used directly as antigen. Mouse polyclonal and monoclonal antibodies were obtained by standard procedures (Harlow and Lane, Antibodies. A Laboratory Manual, Cold Spring Harbor Laboratory Press, Plainview, NY (1998)). Three mice were inoculated with 50 μg of the inclusion body fraction (in complete Freund's adjuvant), then boosted every two weeks with 50 μg of inclusion body fraction in incomplete Freund's adjuvant. All three mice showed robust immune responses; one was sacrificed for hybridoma fusions. Of 800 candidate hybridoma lines, 17 showed a response in ADF1 dot-blot analyses. Subsequent evaluation of the 17 lines by western blotting and immunochemical assays led to the isolation of ten monoclonal lines, including MAb ADF1-8 and MAb ADF1-17.

EXAMPLE 5 Western Blot Analysis of ADF-1 Protein Levels In Mutant *nal*[Pl] and Wildtype Flies.

Frozen heads or bodies were isolated as previously described (Yin et al., Cell, 79:49-58 (1994)), and extracts were prepared by homogenizing 100 μl of frozen head powder in 500 μl of RIPA buffer. Protein concentrations were determined by the Bio-Rad protein assay. Protein samples were denatured in standard loading dye, separated by SDS-PAGE and transferred electrophoretically at 100 mA for 2 hours to a nitrocellulose membrane (Bio-Rad). Each membrane was blocked overnight at 4°C in PBST + 5% milk, then incubated for 1-2 hours with primary antibody in PBST + 5% milk. Primary antibodies used were mouse polyclonal sera against *adf1* (1:1000), mouse monoclonal supernatant (MAb Adf1-17) against *adf1* (1:20), mouse monoclonal supernatant against TBP (1:5), and mouse monoclonal ascites against a-tubulin (1:50,000) (Sigma). The membrane was washed extensively in PBST and incubated for 1-2 hours with HRP-conjugated anti-IgG secondary antibody (Bio-Rad; 1:500). Following extensive washing in PBST, the conjugated products were visualized by enhanced chemiluminescence (Pierce SuperSignal ULTRA Substrate) and autoradiography.

Relative to control levels of two other proteins (TATA-binding protein and alpha-TUBULIN), ADF1 expression was reduced at least two-fold in mutant flies.

EXAMPLE 6 Comparison of DNA Chip Data Sets Between Wildtype Flies and A Single-Gene Mutant, *nalyot.*

DNA chip data sets between normal (wildtype) flies and a single-gene mutant, *nalyot* were compared. The *nalyot* mutant was shown to have normal memory after massed training, but LTM was not induced by spaced training. Moreover, the *nalyot* mutation was identified as a transposon insertion in the *Adf1* gene, the effect of which is to reduce the amount of *Adf1* transcript and protein in mutant fly heads.

When all baseline values were set to zero and the data then were analyzed, no significant difference between wildtype flies and *nalyot* mutants was detected for the *Adf1* gene. However, when baseline values of 10 or less were eliminated from the analysis (rather than set to zero), a significant effect on *Adf1* transcription was detected, which corroborated the results obtained by Northern analysis, as described in Example 3. This result constitutes a significant verification for the statistical approach in which average difference values below 10 are deleted from the data set.

EXAMPLE 7 Quantitative Polymerase Chain Reaction.

A given group of flies is subjected to a particular training protocol (spaced or massed) and stored in regular food vials after training. Twenty four hours after training, flies from different groups of a given training protocol (spaced or massed) are collected into a single 50 ml Falcon tube and quick-frozen in liquid nitrogen. The heads of frozen flies are separated from their bodies by vigorous mechanical shaking. Frozen and separated body parts then are sifted through a series of sieves, ultimately to obtain a homogeneous population of fly heads.

Combined heads from a training group (spaced or massed) are separated into eight groups. Each group of heads then is ground into a powder with mortar and pestal. The powder is transferred to 5 ml of Trizol solution (Gibco) and stored at -70°C overnight.

The frozen Trizol/ fly powder solution then is thawed. 2 ml of chloroform is added. The mixture is centrifuged at 3,500 rpm for 10 minutes at 4°C. The extracted RNA (in aqueous layer) is decanted to a fresh tube, and 1.4 ml of isopropanol is added.

For QPCR, aliquots of the above solution are spun at 8,000 rpm for 20 minutes at 4°C. Isopropanol is decanted, and the pellet is washed 1x in 70% ethanol. The pellet is resuspended in 100 $\mu$l of $H_2O$ and an equal volume of phenol/ chloroform (Gibco) is added. The solution is centrifuged at 14,000 rpm for 5 minutes at 4°C. The top aqueous layer is decanted to a fresh tube. 200 $\mu$l of ice-cold ethanol is added, along with 6 $\mu$l of 3 M sodium acetate. The solution is incubated at -20°C for at least 20 minutes.

The solution is then centrifuged at 14,000 rpm for 20 minutes at 4°C. The pellet is resuspended in 20 $\mu$l of $H_2O$ and subjected to RQ1 Rnase free DNAse (Promega). RNA concentration is determined.

First-strand cDNAs then are synthesized from 1 $\mu$g of the DNAse-free RNA samples, and the QPCR assay is performed according to Perkin Elmer Biosystems protocols, using the 7700 ABI Prism with "CYBR" cybergreen flourescence detection.

EXAMPLE 8 Statistical Candidate Memory Gene, C/EBP.

The following C/EBP primers, designed by Quantagene (Paris, France), were used in QPCR experiments: 5'-AGACTACCGATGCGAACAAC-3' (SEQ ID NO:1) and 3'-GTCCCTGAACTGGTCGTCTA-5' (SEQ ID NO:2), yielding an expected fragment of 221 bp in size.

For C/EBP data, 8 replicated RNA extractions were obtained from approximately 10,000 heads of flies 24 hours after they were subjected to spaced or to massed training. QPCR reactions for each RNA extraction were run in triplicate in accordance with the method outlined in Example 7.

Results for each C/EBP reaction were normalized for RNA amounts against a paired QPCR reaction for TBPßß, a control gene which shows no transcriptional changes in these contexts.

Analyzed this way, the mean number of cycles required for C/EBP amplification to reach the critical value was 21.86 $\pm$ 0.56 for the spaced group and 23.78 $\pm$ 0.56 for the massed group. It took fewer amplification cycles for the spaced group to reach the critical value, indicating a higher concentration of C/EBP at the beginning of the PCR amplification.

These data indicate C/EBP levels were 3.78-fold higher in the spaced training group than in the massed training group (Figure 5). The experimental results confirmed that there is a significant difference for C/EBP (called *slow border cells, slobo* in Drosophila) transcripts isolated from spaced- versus massed-trained flies. Genetic manipulation of C/EBP in mice enhances long-term memory (Sterneck et al., Proc. Natl. Acad Sci. USA, 95(18):10908-10913 (1998)), and molecular manipulation of C/EBP in Aplysia blocks long-term facilitation (a cellular substrate for long-term memory of sensitization) (Alberini et al., Cell, 76:1099-1114 (1994)). Therefore, confirmation of C/EBP as a CMG herein constitutes a significant verification that this DNA chip approach can be used in identifying transcripts involved in memory.

EXAMPLE 9 Training Apparatus.

Experimentally naïve male Sprague-Dawley rats (300-350 g) were trained and tested in five identical Plexiglas and wire-mesh cages (8 × 15 × 15 cm) housed in a sound-atrenuated chamer (Cassella and Davis, Physio. Behav., 36:377-383 (1986)). The startle-eliciting stimulus was a 105 dB, 50-ms burst of white noise (rise-decay time 5 ms), against a background white noise of 55 dB. A 3.7-s light CS was produced by an 8-W fluorescent lightbulb (rise-decay time, 100 $\mu$s; BCO foot-lamberts intensity). The floor on each cage consisted of four stainless steel bars, through which a 0.5-s 0.6 or 0.3 mA scrambled foot shock could be delivered.

EXAMPLE 10 Behavioral Training Procedures.

On each of two days before all training, rats were placed in the startle chambers and 15 minutes later presented with 15 startle stimuli. On the single training day for Exp. 1, animals were placed in the startle chamber and 5 minutes later received 4 light-shock pairings with one of the following intertrial intervals (ITIs) (3-seconds, 5-seconds, 10-seconds, 15-seconds, 2-minutes or 8-minutes). Five minutes following the last light-shock pairing, animals were returned to their home cages. Paired massed and spaced training were similarly conducted except that the ITI between the 4 light-shock pairings was 10 seconds and 8 minutes, respectively. Explicitly unpaired massed training trials consisted of massed presentations of the light (4, ITI of 10 seconds) followed 4 minutes later by massed presentations of the shock (4, ITI of 10 seconds).

EXAMPLE 11 Long Term Memory Testing.

Forty-eight hours following training, rats were placed in the startle apparatus and received 30 startle-eliciting stimuli alone followed by 60 startle-eliciting stimuli, half of which occurred 3.2 seconds after the onset of the 3.7 seconds light (Light-Noise trial) and half of which were presented in darkness (Noise Alone trial). The order of the two trial types was irregular. All startle stimuli were presented at an interstimulus interval of 30 seconds. Fear-potentiated startle difference scores, used as an index of LTM, were calculated by subtracting the average startle amplitudes produced by the 30 Noise Alone trials from the average startle amplitudes produced by the 30 Light-Noise trials.

EXAMPLE 12 Short Term Memory Testing.

Short term memory (STM) testing was similar to LTM testing except that it occurred 15 or 40 minutes following training. Twenty Noise Alone stimuli were followed by 15 Light-Noise stimuli and 15 Noise Alone stimuli intermixed. Fear-potentiated startle scores were calculated by subtracting the average Noise Alone score from the average Light-Noise score and used as an index of STM.

EXAMPLE 13 Surgery,

Rats were pre-treated with atropine sulfate (0.4 mg/kg, ip), anesthetized with sodium pentobarbital (60 mg/kg, ip) and placed in a standard stereotaxic instrument. A Hamilton microsyringe (10 $\mu$l) mounted in an infusion pump was used for infusions. Bilateral microinjections (2 $\mu$l) were delivered over 10 minutes through a 30 gauge cannulae aimed at the lateral nucleus of the amygdala (coordinates AP = - 2.8, L = $\pm$5.2, DV = - 8.5 below the surface of the skull) or caudate nucleus (coordinates AP = +0.2, L = $\pm$3.0, DV = - 6.0) according to Paxinos and Watson, The Rat Brain in Stereotaxic Coordinates, Academic, Syndney, Australia (1986). Infusion cannulae were left in place an additional 10 minutes to ensure diffusion.

EXAMPLE 14 Virus Preparation.

CREB and mCREB cDNAs (obtained from M.E. Greenberg, Harvard University) and LacZ were inserted into the HSV amplicon HSV-PrpUC and packed using the helper 5dl 1.2 (Lim et al., Biotechniques, 20:460-469 (1996); Keve et al., Neuroscience, 79:435-447 (1997)). Virus was purified on a sucrose gradient; pelleted and resuspended in 10% sucrose. The average titer of the recombinant virus stocks was 4.0 x 10' infectious units/ml and was similar for HSV-CREB and HSV-mCREB. Transgene expression was regulated by the constitutive promoter for the HSV immediate-early gene IE 4/5.

EXAMPLE 15 Immunochemistry.

Rats were overdosed with chloral hydrate and perfused with 50 ml PBS followed by 250 ml 4% paraformaldehyde in PBS. The brains were cryoprotected and cut on a microtome (40 $\mu$m sections), and immunocytochemistry was performed on free-floating sections. Brains infected with HSV-LacZ were reacted for β-galactosidase and counterstained with neutral red (according to Lim et al., Biotechniques, 20:460-469 (1996); Keve et al., Neuroscience, 79:435-447 (1997)). Briefly, to detect β-galactosidase activity, brain slices were allowed to react for 2 hours in a solution comprised of 3.1 mM potassium ferrocyanide, 3.1 mM potassium ferricyanide, 20 mM MgCl$_2$, 0.1 M PBS and 0.2 mg/ml X-gal (Boehringer-Mannheim).

Analysis of transgene expression in brain infected with HSV-CREB was conducted. Briefly, sections were incubated with 1% H$_2$O$_2$ and 0.3% Triton-X for 20 minutes, blocked with 1% bovine serum albumin, 2% normal goat serum and 0.3% Titron-X for 30 minutes and incubated with the primary antibody, CREB (1:1000; Upstate Biotechnology, Lake Placid, NY) overnight at 4°C with constant agitation. Sections were incubated with biotinylated goat-anti rabbit IgG secondary antiserum (1:200 dilution; Vector Laboratories, Burlingame, CA) for 2 hours at room temperature. Sections were rinsed and incubated with avidin-biotin peroxidase complex (ABC) reagent (Vector Laboratories). Immunoreactivity was visualized using diaminobenzidine (DAB) reaction.

EXAMPLE 16 Effect of Intertrial interval Between Fear Conditioning Trials On Subsequent Levels of Long Term Memory.

Mean LTM ($\pm$ SEM; assessed as fear-potentiated startle difference scores), assessed 48 hours following training that consisted of 4 light-shock pairings with ITis of 3 seconds, 5 seconds, 10 seconds, 15 seconds, 2 minutes and 8 minutes (n=10, 10, 10, 5, 5, 15, respectively), varied with different ITIs ($F_{1,49}$= 3.04, p < 0.05). The level of LTM is a linear function of the ITI with longer ITIs producing more robust LTM as shown by a significant linear trend ($F_{1,49}$ = 7.99, p < 0.05). Massed training (3 seconds, 5 seconds, 10 seconds) produces very weak LTM (roughly 50 units). Increasing the rest interval from 10 seconds to 8 minutes yields an increase in LTM as post-hoc Duncan comparisons reveals that 8 min ITI (spaced) produced significantly greater LTM than 10 seconds, 5 seconds and 3 seconds (massed). The results are depicted in Figure 6.

EXAMPLE 17 Effect Of Infusion of HSV Vectors Into the Basolateral Amygdala and Extra-amygdala Areas.

Rats infused with HSV-CREB into the basolateral amygdala (n=17) showed significantly greater LTM than rats similarly infused with PBS (n=7), HSV-LacZ (n=10), or HSV-mCREB (n=11) or rats infused with HSV-CREB into brain regions surrounding the basolateral complex of the amygdala (n=8) or into a control region (the caudate; n=5) ($F_{5,52}$ = 4.99, p < 0.001). Post-hoc analysis revealed that the level of LTM in rats that received HSV-CREB infusion into the basolateral amygdala was significantly higher than all other groups.

Reactivity to footshock was not different for animals given HSV-CREB (n=17), HSV-mCREB (n=11), HSV-LacZ (n=10) or PBS (n=7) infusion into the basolateral amygdala prior to massed training ($F_{3,41}$ =1.41, p > 0.05). Mean shock reactivity was assessed by cage displacement for the 200-ms period after each of the 4 footshocks.

Explicitly unpaired conditioning (in which CS (conditioned stimulus) and US (unconditioned stimulus) are not associated) failed to produce LTM for the association in control rats (n=10) and intra-amygdala infusion of PBS (n=5) or HSV-CREB (n=5) did not change this ($F_{2,17}$ = 0.44, p > 0.05).

Animals that received HSV-CREB 3 days prior to massed training (3d HSV-CREB, n=10) showed greater LTM when re-tested 14 days following infusion than animals similarly treated with HSV-LacZ (3d HSV-LacZ, n=3) or animals given HSV-CREB 14 days prior to massed training and tested 48 h later (14d HSV-CREB, n=4) ($F_{2,14}$ = 6.05, p < 0.05).

**Claims**

1.  A method identifying a candidate gene or genes involved in transcription dependent memory of associative learning comprising the steps of:

    (a) training non-human animals to induce transcription-dependent memory formation in said animals;
    (b) extracting RNA from brain tissue of said animals trained in step (a);
    (c) synthesizing DNA probes using the RNA extracted in step (b);
    (d) hybridizing the DNA probes synthesized in step (c) to complementary DNA sequences on microarray chips containing DNA sequences from genes of the genome of said animals, wherein a signal is produced upon hybridization of said probes to complementary DNA sequences;

(e) detecting the signal produced in step (d); and

(f) performing a statistical comparison between the signal detected in step (e) and the signal detected in a control for each gene using a signal transformation algorithm,

wherein said control is obtained according to a method comprising the steps of:

(i) extracting RNA from brain tissue of non-human control animals trained to induce transcription-independent memory formation but not transcription-dependent associative memory formation is said control animals;

(ii) synthesizing DNA probes using the RNA extracted in step (f)(i); and

(iii) hybridizing the DNA probes synthesized in step (f)(ii) to complementary DNA sequences on microarray chips containing DNA sequences from genes of the genome of control animals, wherein a signal is produced upon hybridization of said probes to complementary DNA sequences; and

wherein memory formation in the animal of step (a) is induced using a spaced training protocol and memory formation in the control animals is induced using a massed-training protocol; and

both the spaced training protocol and the massed training protocol use pavlovian conditioning procedures with a conditioned stimulus and an unconditioned stimulus that are temporally paired ; and

wherein a statistically significant difference between the signal detected in step (e) and the signal detected in a control for each gene indicates a candidate memory gene involved in transcription-dependent associative memory.

2. The method of Claim 1, wherein said non-human animals are non-human mammals and RNA is extracted from brain tissue of said mammals.

3. The method of claim 2, wherein said brain tissue is amygdala or hippocampus.

4. The method of Claim 1, wherein the Pavlovian conditioning procedure is a contextual fear conditioning training protocol.

5. The method of Claim 1, wherein the protocol used for massed and spaced training induces memory of a specific experimental task.

6. The method of claim 1, wherein the Pavlovian conditioning procedure is olfactory Pavlovian conditioning.

7. The method of Claim 6, wherein said non-human animals are Drosophila and RNA is extracted from head tissue of said Drosophila.

**Patentansprüche**

1. Verfahren zur Identifizierung eines Kandidaten-Gens oder von Kadidaten-Genen, die am transkriptionsabhängigen Gedächtnis des assoziativen Lernens beteiligt sind, das die folgenden Schritte umfasst:

(a) Trainieren von nichthumanen Tieren zur Induktion transkriptionsabhängiger Gedächtnisbildung in den Tieren;

(b) Extrahieren von RNA aus Himgewebe der in Schritt (a) trainierten Tiere;

(c) Synthetisieren von DNA-Sonden unter Verwendung der in Schritt (b) extrahierten RNA;

(d) Hybridisierung der in Schritt (c) synthetisierten DNA-Sonden mit komplementären DNA-Sequenzen auf Mikroarray-Chips, die DNA-Sequenzen aus Genen des Genoms der Tiere enthalten, wobei ein Signal bei Hybridisierung der Sonden mit komplementären DNA-Sequenzen erzeugt wird; und

(e) Nachweis des in Schritt (d) erzeugten Signals; und

(f) Durchführen eines statistischen Vergleichs zwischen dem in Schritt (e) nachgewiesenen Signal und dem in einer Kontrolle nachgewiesenen Signal für jedes Gen unter Verwendung eines Signal-Transformationsalgorithmus,

wobei die Kontrolle nach einem Verfahren erhalten wird, das die folgenden Schritte umfasst:

(i) Extrahieren von RNA aus Himgewebe nicht-humaner Kontrolltiere, trainiert zur Induktion transkriptionsunabhängiger Gedächtnisbildung aber nicht transkriptionsabhängiger Assoziativgedächtnisbildung in den Kontrolltieren;

(ii) Synthetisieren von DNA-Sonden unter Verwendung der in Schritt (f)(i) extrahierten RNA; und

(iii) Hybridisieren der in Schritt (f)(ii) synthetisierten DNA-Sonden mit komplementären DNA-Sequenzen auf Mikroarray-Chips, die DNA-Sequenzen aus Genen des Genoms von Kontrolltieren enthalten, wobei ein Signal bei Hybridisierung der Sonden mit komplementären DNA-Sequenzen erzeugt wird; und

wobei die Gedächtnisbildung in den Tieren gemäß Schritt (a) induziert ist unter Verwendung eines Wiederholungstrainingsprotokolls mit Unterbrechungen ("spaced training protocol") und die Gedächtnisbildung in den Kontrolltieren induziert ist durch ein unterbrechungsloses Wiederholungstraining ("massed training protocol"); und

sowohl das Wiederholungstrainingsprotokoll mit Unterbrechungen als auch das unterbrechungslose Wiederholungstraining Pawlow'sche Konditionierungsverfahren nutzt mit einer bedingtem Stimulierung und einer bedingungslosen Stimulierung, die temporär gepaart sind; und

wobei ein statistisch signifikanter Unterschied zwischen dem detektierten Signalen in Schritt (e) und dem Signal detektiert in einer Kontrolle für jedes Gen ein Kandidaten-Gedächtnis-Gen anzeigt, involviert in das transkriptionsabhängige Assoziativ-Gedächtnis.

2. Verfahren nach Anspruch 1, wobei die nichthumanen Tiere nichthumane Säuger sind und RNA aus dem Hirngewebe der Säuger extrahiert wird.

3. Verfahren nach Anspruch 2, wobei das Hirngewebe Amygdala oder Hippocampus ist.

4. Verfahren nach Anspruch 1, wobei das Pawlow'sche Konditionierung-Verfahren ein Kontext-Furcht-Konditionierungstrainingsprotokoll ist.

5. Das Verfahren nach Anspruch 1 wonach das für das unterbrechungslose Wiederholungstraining und das Wiederholungstrainingsprotokoll mit Unterbrechungen verwendete Protokoll das Gedächtnis an eine spezielle experimentelle Aufgabe induziert.

6. Das Verfahren nach Anspruch 1 wobei das Pawlow'sche Konditionierungsverfahren ein olfaktorisches Pawlow'sches Konditionierung-Verfahren ist.

7. Verfahren nach Anspruch 6, wobei die nicht-humanen Tiere Drosophila sind und RNA aus dem Kopfgewebe der Drosophila extrahiert wird.

**Revendications**

1. Procédé d'identification d'un ou de gènes candidats impliqués dans la mémoire dépendante de la transcription de l'apprentissage associatif comprenant les étapes de :

(a) entraînement d'animaux non humains pour induire la formation d'une mémoire dépendante de la transcription chez lesdits animaux ;

(b) extraction de l'ARN du tissu cérébral desdits animaux entraînés dans l'étape (a) ;

(c) synthèse de sondes d'ADN en utilisant l'ARN extrait dans l'étape (b) ;

(d) hybridation des sondes d'ADN synthétisées dans l'étape (c) aux séquences d'ADN complémentaire sur des puces à ADN contenant les séquences d'ADN de gènes du génome desdits animaux, dans lequel un signal est produit après l'hybridation desdites sondes aux séquences d'ADN complémentaire ;

(e) détection du signal produit dans l'étape (d) ; et

(f) réalisation d'une comparaison statistique entre le signal détecté dans l'étape (e) le signal détecté dans un témoin pour chaque gène en utilisant un algorithme de transformation de signal,

dans lequel ledit témoin est obtenu selon un procédé comprenant les étapes de :

(i) extraction de l'ARN du tissu cérébral d'animaux témoins non-humains entraînés pour induire la formation d'une mémoire indépendante de la transcription mais pas la formation d'une mémoire dépendante de la transcription chez lesdits animaux témoins ;

(ii) synthèse de sondes d'ADN en utilisant l'ARN extrait dans l'étape (f)(i) ; et

(iii) hybridation des sondes d'ADN synthétisées dans l'étape (f)(ii) aux séquences d'ADN complémentaire sur des puces à ADN contenant les séquences d'ADN de gènes du génome des animaux témoins, dans lequel un signal est produit après l'hybridation desdites sondes aux séquences d'ADN complémentaire ; et

dans lequel la formation de la mémoire chez les animaux de l'étape (a) est induite en utilisant un protocole d'apprentissage espacé et la formation de la mémoire chez les animaux témoins est induite en utilisant un protocole d'apprentissage massé ; et
à la fois le protocole d'apprentissage espacé et le protocole d'apprentissage massé utilisent des procédures de conditionnement pavlovien avec un stimulus conditionné et un stimulus non conditionné qui sont associés de manière temporaire ; et
dans lequel une différence statistique significative entre le signal détecté à l'étape (e) et le signal détecté chez un témoin pour chaque gène indique un gène mémoire candidat impliqué dans la mémoire associative dépendante de la transcription.

2. Procédé selon la revendication 1, dans lequel lesdits animaux non-humains sont des mammifères non-humains et l'ARN est extrait du tissu cérébral desdits mammifères.

3. Procédé selon la revendication 2, dans lequel ledit tissu cérébral est l'amygdale ou l'hippocampe.

4. Procédé selon la revendication 1, dans lequel la procédure de conditionnement pavlovien est un protocole d'entraînement à un conditionnement de peur contextuelle.

5. Procédé selon la revendication 1, dans lequel le protocole utilisé pour l'apprentissage massé et espacé induit la mémoire d'une tâche expérimentale spécifique.

6. Procédé selon la revendication 1, dans lequel la procédure de conditionnement pavlovien est un conditionnement pavlovien olfactif.

7. Procédé selon la revendication 6, dans lequel lesdits animaux non-humains sont des Drosophiles et l'ARN est extrait du tissu de la tête desdites Drosophiles.

TRAINING

odor 1    odor 2

shock

TESTING

odor 1 vs. odor 2

FIG. 1

EP 1 571 226 B1

FIG. 2

# Calculation of gene-expression level with Affy chips: 20 primer pairs per gene

## Average difference between PM and MM signal

PM

MM

Fig. 3

Fig. 4A

Fig. 4B

EP 1 571 226 B1

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9849342 A
- US 5445934 A

**Non-patent literature cited in the description**

- **Cavallaro S et al.** *PNAS,* 1997, vol. 94, 9669-9673
- **Tully et al.** *Society for Neuroscience abstracts,* 1993, vol. 19 (1/3), 1066
- **Tully et al.** *Cell,* 1994, vol. 79, 35-47
- **Yin et al.** *Cell,* 1994, vol. 79, 49-58
- **Yin et al.** *Cell,* 1995, vol. 81, 107-115
- Molecular Cloning: A Laboratory Manual. Cold Spring Harbor University Press, 1989
- Current Protocols In Molecular Biology. John Wiley & Sons, 1997
- **Drain et al.** *Neuron,* 1991, vol. 6, 71-82
- **Tully ; Quinn.** *J. Comp. Physiol.,* 1985, vol. 157, 263-277
- **Ramsay.** *Nature Biotechnology,* 1998, vol. 16, 40-44
- **Dubnau ; Tully.** *Ann. Rev. Neurosci.,* 1998, vol. 21, 407-444
- **Josselyn et al.** *Society for Neurosci.,* 1998, vol. 24, 926
- Molecular Clowning: A Laboratory Manual. Cold Spring Harbor University Press, 1989
- **Cassella ; Davis.** *Physiol. Behav.,* 1986, vol. 36, 377-383
- **Guzowski et al.** *Proc. Natl. Acad Sci. USA,* 1997, vol. 94, 2693-2698
- **Lamprecht et al.** *J. Neuroscience,* 1997, vol. 17 (21), 6443-6450
- **Bourtchuladze et al.** *Cell,* 1994, vol. 79, 59-68
- **Kogan et al.** *Curr. Biol.,* 1996, vol. 7, 1-11
- **Taubenfeld et al.** *Nat. Neurosci.,* 1999, vol. 2 (4), 309-310
- **Imprey et al.** *Nat. Neurosci.,* 1998, vol. 1 (7), 595-601
- **Bier et al.** *Science,* 1988, vol. 240 (4854), 913-916
- **Boynton ; Tully.** *Genetics,* 1992, vol. 131, 655-672
- **Dura et al.** *J. Neurogent,* 1993, vol. 9, 1-14
- **Freeman et al.** *Development,* 1999, vol. 126, 4591-4602
- **Pinto et al.** *Neuron,* 1999, vol. 23, 45-54
- **Rohrbough et al.** *Neuron,* 1999, vol. 23, 55-70
- **Bolwig et al.** *Neuron,* 1995, vol. 15, 829-842
- **Simon et al.** *Mech. Dev.,* 1998, vol. 76, 42-55
- **England et al.** *Proc. Natl. Acad. Sci. USA,* 1992, vol. 89, 683-687
- **Harlow ; Lane.** Antibodies. A Laboratory Manual. Cold Spring Harbor Laboratory Press, 1998
- **Sterneck et al.** *Proc. Natl. Acad Sci. USA,* 1998, vol. 95 (18), 10908-10913
- **Alberini et al.** *Cell,* 1994, vol. 76, 1099-1114
- **Cassella ; Davis.** *Physio. Behav.,* 1986, vol. 36, 377-383
- **Paxinos ; Watson.** The Rat Brain in Stereotaxic Co-ordinates. Academic, 1986
- **Lim et al.** *Biotechniques,* 1996, vol. 20, 460-469
- **Keve et al.** *Neuroscience,* 1997, vol. 79, 435-447